# FASCICULE DE BREVET EUROPEEN

(11) **EP 2 784 066 B1**
(45) Date de publication et mention de la délivrance du brevet: **01.07.2015**
(21) Numéro de dépôt: 14161388.5
(22) Date de dépôt: 25.03.2014
(51) Int. Cl.: C07C 235/34

(54) **Procédé de synthèse de dérivés de la 7,8-dimethoxy-1,3-dihydro-2H-3-benzazépin-2-one et application à la synthèse de l'ivabradine**
Verfahren zur Herstellung von Derivaten von 7,8-dimethoxy-1,3-dihydro-2H-3-benzazepin-2-on und ihre Verwendung zur Herstellung von Ivabradin
Process for the synthesis of derivatives of 7,8-dimethoxy-1,3-dihydro-2H-3-benzazepin-2-one and its use for the preparation of ivabradine

(30) Priorité: 26.03.2013 FR 1352741
(43) Date de publication de la demande: 01.10.2014
(73) Titulaire: Les Laboratoires Servier, 92284 Suresnes Cedex (FR)
(72) Inventeur: Le Flohic, Alexandre, 76640 Fauville en Caux (FR)

(56) Documents cités:
- EP-A1- 0 534 859
- WO-A2-2008/065681

## Description

La présente invention concerne un procédé de synthèse du composé de formule (I): 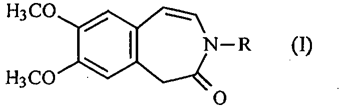 dans laquelle R représente un groupement *para*-méthoxybenzyle (PMB) ou le groupement suivant : 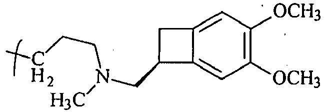 ainsi que l'application de ce procédé de synthèse à la préparation de l'ivabradine et de l'un de ses intermédiaires clés de synthèse.

L'ivabradine de formule (II) : 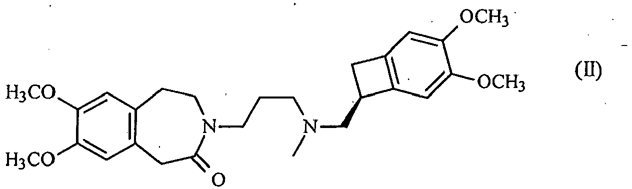 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrites dans le brevet européen EP 0 534 859.

Ce brevet décrit la synthèse du chlorhydrate de l'ivabradine à partir du composé de formule (III) : 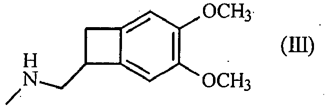 qui est dédoublé pour conduire au composé de formule (IV) : 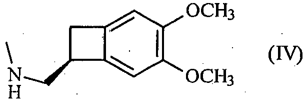 qui est mis en réaction avec le composé de formule (V) : 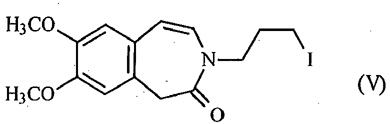 pour conduire au composé de formule (VI) : 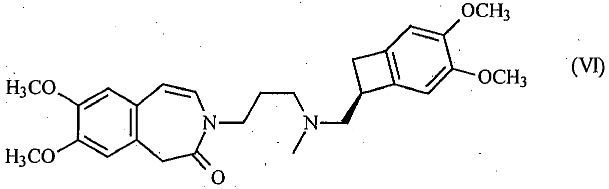 dont l'hydrogénation catalytique conduit à l'ivabradine, qui est alors transformée en son chlorhydrate.

L'inconvénient de la voie de synthèse décrite dans EP 0 534 859 est de ne conduire à l'ivabradine qu'avec un rendement de 1%.

Le brevet EP 0 534 859 décrit également la préparation du composé de formule (V), intermédiaire de synthèse de l'ivabradine, à partir de la 3-(3-chloropropyl)-7,8-diméthoxy-1,3-dihydro-2*H*-3-benzazépin-2-one.

Ce brevet renvoie, pour la préparation de la 3-(3-chloropropyl)-7,8-diméthoxy-1,3-dihydro-2*H*-3-benzazépin-2-one, à la publication de Reiffer M. et al. (J. Med. Chem. 1990 ; vol. 33 (5), pages 1496-1504). Cette publication décrit la synthèse de ce dérivé chloré à partir de la 7,8-diméthoxy-1,3-dihydro-2*H*-3-benzazépin-2-one.

Compte-tenu de l'intérêt pharmaceutique de l'ivabradine, il est important de pouvoir y accéder avec un procédé de synthèse performant, présentant un bon rendement.

Il est également particulièrement intéressant d'accéder avec de bons rendements aux dérivés de 7,8-diméthoxy-1,3-dihydro-2*H*-3-benzazépin-2-one, précurseurs de l'ivabradine.

La présente invention concerne un procédé de synthèse du composé de formule (I): 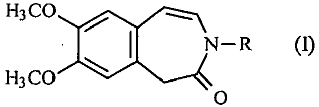 dans laquelle R représente un groupement *para*-méthoxybenzyle (PMB) ou le groupement suivant : 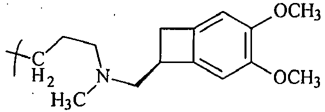 caractérisé en ce que le composé de formule (VII): 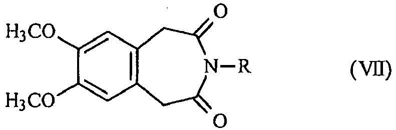 dans laquelle R est tel que défini précédemment,
est soumis à une réaction de réduction,
en présence de LiBH(Et)₃,
dans un solvant organique,
pour conduire au composé de formule (I).

La quantité de LiBH(Et)₃ préférentiellement utilisée pour effectuer la réaction de réduction du composé de formule (VII) en composé de formule (I) est comprise entre 1 et 3 équivalents.

Parmi les solvants organiques pouvant être utilisés pour effectuer la réaction de réduction du composé de formule (VII) en composé de formule (I), on peut citer à titre non limitatif le tétrahydrofurane (THF), le méthyl-tétrahydrofurane (Me-THF), le dichlorométhane, le toluène ou le diisopropyl éther.

Le solvant organique préférentiellement utilisé pour effectuer la réaction de réduction du composé de formule (VII) en composé de formule (I) est le tétrahydrofurane.

La réaction de réduction du composé de formule (VII) en composé de formule (I) est préférentiellement conduite à une température comprise entre -100 °C et 20°C.

Dans le cas où R représente le groupement suivant : 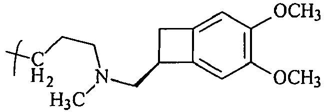 la présente invention concerne également un procédé de synthèse de l'ivabradine,
caractérisé en ce que le composé de formule (VIII), cas particulier des composés de formule (VII) : 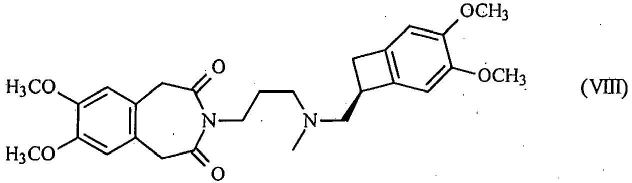 est soumis à une réaction de réduction selon le procédé décrit plus haut,
pour conduire au composé de formule (VI), cas particulier des composés de formule (I) : 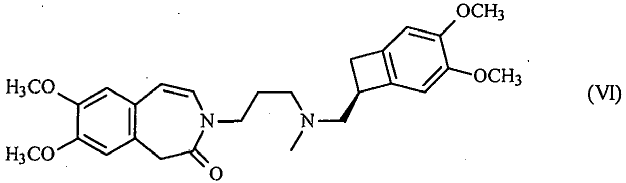 puis le composé de formule (VI) subit une hydrogénation catalytique pour conduire à l'ivabradine de formule (II) : 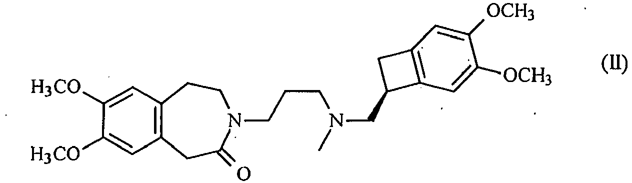 qui peut être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

La présente invention concerne également un procédé de synthèse de l'ivabradine à partir du composé de formule (VIII), caractérisé en ce que ledit composé de formule (VIII) est préparé à partir du composé de formule (IX) : 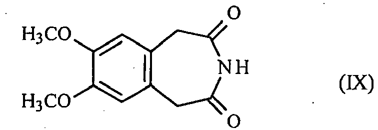 qui est mis en réaction avec le composé de formule (X) : 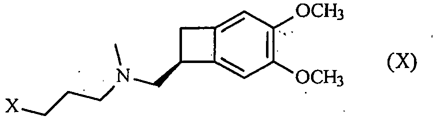 dans laquelle X représente un atome d'halogène, un groupement mésylate ou un groupement tosylate,
en présence d'une base,
dans un solvant organique,
pour conduire au composé de formule (VIII) : 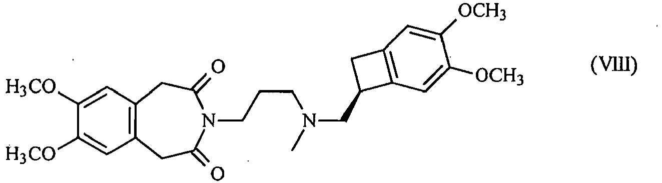 lequel est transformé en composé de formule (VI) selon le procédé décrit plus haut,
ledit composé de formule (VI) étant transformé en ivabradine de formule (II) : 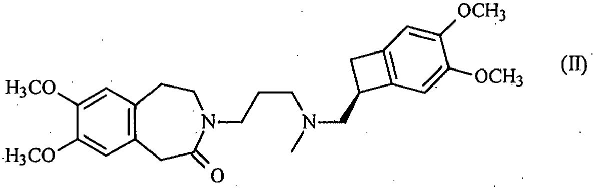 par la réaction d'hydrogénation catalytique décrite plus haut.

Parmi les bases pouvant être utilisées pour effectuer la réaction entre le composé de formule (IX) et le composé de formule (X), on peut citer à titre non limitatif des bases inorganiques comme le carbonate de potassium, le carbonate de sodium, le carbonate de césium, l'hydrogénocarbonate de potassium et l'hydrogénocarbonate de sodium, et des bases organiques comme la triéthylamine, la diisopropyléthylamine et la pyridine.

La base préférentiellement utilisée pour effectuer la réaction entre le composé de formule (IX) et le composé de formule (X) est le carbonate de potassium.

Parmi les solvants organiques pouvant être utilisés pour effectuer la réaction entre le composé de formule (IX) et le composé de formule (X), on peut citer à titre non limitatif l'acétonitrile, l'acétone, la méthyléthylcétone (MEK), le diméthylformamide (DMF), la *N-*méthylpyrrolidone (NMP) et le diméthylsulfoxyde (DMSO).

Le solvant organique préférentiellement utilisé pour effectuer la réaction entre le composé de formule (IX) et le composé de formule (X) est le diméthylformamide (DMF).

La réaction entre le composé de formule (IX) et le composé de formule (X) est préférentiellement conduite à une température comprise entre 20 °C et 150°C.

La présente invention concerne également un procédé de synthèse de l'ivabradine à partir du composé de formule (VIII), caractérisé en ce que ledit composé de formule (VIII) est préparé à partir du composé de formule (XI) : 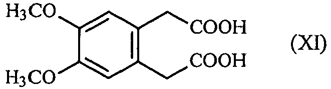 qui est transformé en composé de formule (XII) : 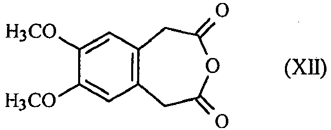 par une réaction de cyclisation en présence d'un agent de couplage,
dans un solvant organique,
ledit composé de formule (XII) étant ensuite mis en réaction avec le composé de formule (XIII) : 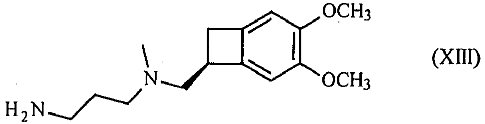 en présence d'une base,
dans un solvant organique,
pour conduire au composé de formule (XIV) : 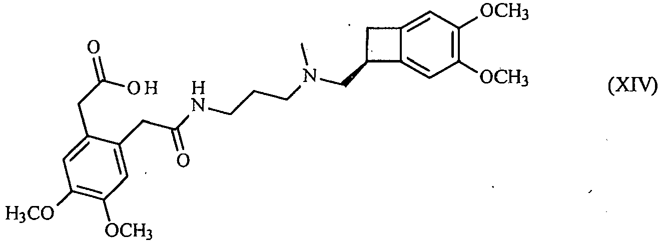 qui est soumis à une réaction de cyclisation en présence d'un agent de couplage,
dans un solvant organique
pour conduire au composé de formule (VIII) : 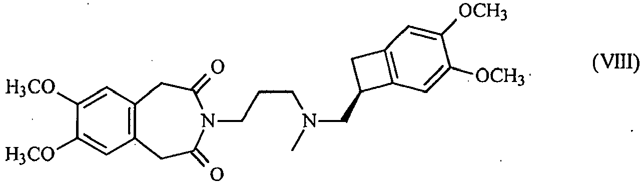 lequel est transformé en composé de formule (VI) selon le procédé décrit plus haut,
ledit composé de formule (VI) étant transformé en ivabradine de formule (II) : 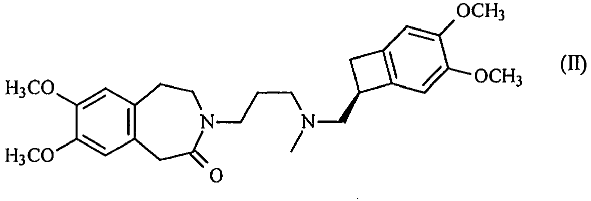 par la réaction d'hydrogénation catalytique décrite plus haut.

Le composé de formule (XII) est préférentiellement formé *in situ,* c'est-à-dire qu'il n'est pas isolé avant d'être mis en réaction avec le composé de formule (XIII).

Parmi les agents de couplage pouvant être utilisés pour la réaction de cyclisation du composé de formule (XI) en composé de formule (XII), on peut citer à titre non limitatif les réactifs suivants : le chlorure d'oxalyle, le chlorure de thionyle, le *N,N*-dicyclohexyl carbodiimide (DCC), le 1-éthyl-3-(3-diméthylaminopropyl)carbodiimide (EDCI), le *N,N-*carbonyldiimidazole (CDI), l'anhydride cyclique 1-propanephosphonique (T3P) et le 1-(méthylsulfonyl)-1H-benzotriazole.

L'agent de couplage préférentiellement utilisé pour la réaction de cyclisation du composé de formule (XI) en composé de formule (XII) est le chlorure de thionyle.

La quantité de chlorure de thionyle préférentiellement utilisée pour effectuer la réaction de cyclisation du composé de formule (XI) en composé de formule (XII) est comprise entre 1 et 5 équivalents.

Parmi les solvants organiques pouvant être utilisés pour effectuer la réaction de cyclisation du composé de formule (XI) en composé de formule (XII), on peut citer à titre non limitatif le tétrahydrofurane (THF), le méthyl-tétrahydrofurane (Me-THF), le dichlorométhane, le toluène ou le di-iso-propyl éther.

Le solvant organique préférentiellement utilisé pour effectuer la réaction de cyclisation du composé de formule (XI) en composé de formule (XII) est le toluène.

La réaction de cyclisation du composé de formule (XI) en composé de formule (XII) est préférentiellement conduite à une température comprise entre 20 °C et 110 °C.

Parmi les bases pouvant être utilisées pour effectuer la réaction entre le composé de formule (XII) et le composé de formule (XIII), on peut citer à titre non limitatif des bases inorganiques comme le carbonate de potassium, le carbonate de sodium, le carbonate de césium, l'hydrogénocarbonate de potassium et l'hydrogénocarbonate de sodium, et des bases organiques comme la triéthylamine, la diisopropyléthylamine et la pyridine.

La base préférentiellement utilisée pour la réaction entre le composé de formule (XII) et le composé de formule (XIII) est la triéthylamine.

Parmi les solvants organiques pouvant être utilisés pour effectuer la réaction entre le composé de formule (XII) et le composé de formule (XIII), on peut citer à titre non limitatif le tétrahydrofurane (THF), le méthyl-tétrahydrofurane (Me-THF), le dichlorométhane, le toluène ou le diisopropyl éther.

Le solvant organique utilisé pour effectuer la réaction entre le composé de formule (XII) et le composé de formule (XIII) peut également être constitué par un mélange de deux solvants parmi les solvants organiques précédemment cités.

Le solvant organique préférentiellement utilisé pour effectuer la réaction entre le composé de formule (XII) et le composé de formule (XIII) est un mélange de toluène et de dichlorométhane.

La réaction entre le composé de formule (XII) et le composé de formule (XIII) est préférentiellement conduite à une température comprise entre 0 °C et 110°C.

Parmi les agents de couplage pouvant être utilisés pour effectuer la réaction de cyclisation du composé de formule (XIV), on peut citer à titre non limitatif les réactifs suivants : le chlorure d'oxalyle, le chlorure de thionyle, le *N,N*-dicyclohexyl carbodiimide (DCC), le 1-éthyl-3-(3-diméthylaminopropyl)carbodiimide (EDCI), le *N,N*-carbonyldiimidazole (CDI), l'anhydride cyclique 1-propanephosphonique (T3P) et le 1-(méthylsulfonyl)-1H-benzotriazole.

L'agent de couplage préférentiellement utilisé pour effectuer la réaction de cyclisation du composé de formule (XIV) est le chlorure de thionyle.

La quantité chlorure de thionyle préférentiellement utilisée pour effectuer la réaction de cyclisation du composé de formule (XIV) est comprise entre 1 et 3 équivalents.

Parmi les solvants organiques pouvant être utilisés pour effectuer la réaction de cyclisation du composé de formule (XIV), on peut citer à titre non limitatif le tétrahydrofurane (THF), le méthyl-tétrahydrofurane (Me-THF), le dichlorométhane, le toluène ou le diisopropyl éther.

Le solvant organique utilisé pour effectuer la réaction de cyclisation du composé de formule (XIV) peut également être constitué par un mélange de deux solvants parmi les solvants organiques précédemment cités.

Le solvant organique préférentiellement utilisé pour effectuer la réaction cyclisation du composé de formule (XIV) est un mélange de toluène et de dichlorométhane.

La réaction de cyclisation du composé de formule (XIV) est préférentiellement conduite à une température comprise entre 0 °C et 110 °C.

Dans le cas où R représente un groupement *para*-méthoxybenzyle, la présente invention concerne également un procédé de synthèse du composé de formule (XV) : 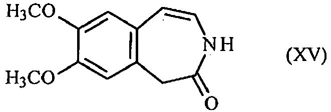 caractérisé en ce que le composé de formule (XVI), cas particulier des composés de formule (VII) : 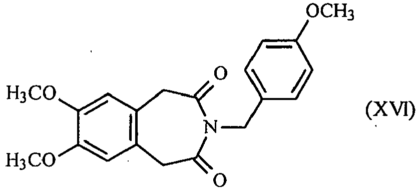 est soumis à une réaction de réduction selon le procédé décrit plus haut,
pour conduire au composé de formule (XVII), cas particulier des composés de formule (I) : 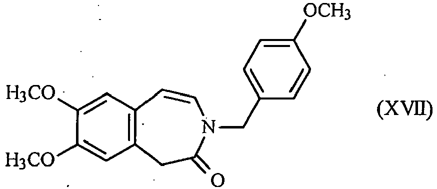 puis le composé de formule (XVII) est déprotégé pour conduire au composé de formule (XV).

La déprotection du composé de formule (XVII) est préférentiellement conduite dans l'acide trifluoroacétique à reflux.

Le composé de formule (XV) est utile comme intermédiaire de synthèse de l'ivabradine, comme cela a été détaillé dans la demande EP 2 135 861.

Les composés de formule (VIII) et (XIV) sont des produits nouveaux, utiles comme intermédiaires de synthèse dans l'industrie chimique ou pharmaceutique, notamment dans la synthèse de l'ivabradine, de ses sels d'addition à un acide pharmaceutiquement acceptable et de leurs hydrates, et font à ce titre partie intégrante de la présente invention.

### Liste des abréviations utilisées :

DMF : diméthylformamide
IR : infrarouge
RMN : Résonance Magnétique Nucléaire
PF : point de fusion
THF : tétrahydrofurane

La chromatographie flash sur colonne de silice est réalisée avec un appareil chromatographique automatisée Buchi Sepacore.

Les spectres RMN sont enregistrés sur un appareil Brüker à 400 MHz pour les spectres proton et à 100MHz pour les spectres carbone.

Les déplacements chimiques sont exprimés en ppm (référence interne TMS).
Les abréviations suivantes ont été utilisées pour qualifier les pics : singulet (s), doublet (d), doublet de doublet (dd), triplet (t), quadruplet (q), multiplet (m).

Les exemples ci-dessous illustrent l'invention.

### PREPARATION A :

### Oxalate de 3-{3-[{[(7S)-3,4-diméthoxybicyclo[4.2.0]octa-1,3,5-trién-7-yl]méthyl} (méthyl)amino]propyl}-7,8-diméthoxy-1H-3-benzazépine-2,4(3H,5H)-dione

5,9 g de 7,8-diméthoxy-1*H*-3-benzazépine-2,4(3*H*,5*H*)-dione (25,1 mmol), 7,1 g de 3-chloro-N- {[(7*S*)-3,4-diméthoxybicyclo[4.2.0]octa-1,3,5-trién-7-yl]méthyl}-*N*-méthylpropan-1-amine (25,1 mmol, 1éq), 1,5 équivalent de K₂CO₃ (37,5 mmol), 0,2 équivalent de KI (5 mmol) et 60 mL de DMF sont introduits dans un réacteur. Le milieu réactionnel est chauffé à 80°C pendant 2h, refroidit à température ambiante puis 90 mL d'eau glacée est introduite. Le produit est extrait par du dichlorométhane (2 x 60 mL). La phase organique est lavée avec une solution aqueuse de NaHCO₃ à 10% (60 mL) puis une solution aqueuse saturée en NaCl jusqu'à élimination du DMF.
Après mise à sec, le produit est obtenu sous forme d'une huile (11,4g, 23,6 mmol). Cette dernière est mise en solution dans l'acétate d'éthyle (34 mL). Le milieu est chauffé au reflux puis on introduit une solution d'acide oxalique (23,6 mmol) dans l'éthanol (34 ml). Le milieu est refroidi à température ambiante, maintenu 2h sous agitation puis filtré et le filtrat est lavé à l'éthanol (20 mL). Le produit salifié est séché en étuve ventilée à 40°C, on obtient 8,4g de produit du titre sous forme d'une poudre beige.

### Rendement : 57%

### Analyse de la base:

*RMN ¹H (CDCl₃, 400MHz)* : 1.59 ppm (2H, m) - 1.61 ppm (2H, m) - 2.16 ppm (3H, m) - 2.39 ppm (1H, m) - 2.60 ppm (3H, m) - 3.13 ppm (1H, m) - 3.22 ppm (2H, m) - 3.28 ppm (1H, m) - 3.36 ppm (1H, m) - 3.66 ppm (3H, s) - 3.67 ppm (3H, s) - 3.68 ppm (3H, s) - 3.69 ppm (3H, s) - 3.71 ppm (2H, m) - 6.64 ppm (1H, d) - 6.68 ppm (1H, d) - 6.71 ppm (1H, s)-6.81 ppm (1H, s).
*RMN ¹³C (CDCl₃, 100MHz)*: 26.24 ppm (CH₂) - 27.55 ppm (CH₂) - 36.38 ppm (CH₂)-39.16 ppm (CH₂)- 41.54 ppm (CH₂) -41.58 ppm (CH) - 42.90 ppm (CH₃) - 56.54 ppm (CH₂) - 56.56 ppm (CH₃) - 56.70 ppm (CH₃) - 56.98 ppm (CH₃) - 57.03 ppm (CH₃) - 63.15 ppm (CH₂) - 108.63 ppm (CH) - 109.30 ppm (CH) - 115.34 ppm (CH) - 115.73 ppm (CH) - 127.08 ppm (Cq) - 128.15 ppm (Cq) - 136.66 ppm (Cq) - 140.30 ppm (Cq) - 149.60 ppm (Cq) - 149.76 ppm (Cq) - 150.99 ppm (Cq) - 151.57 ppm (Cq) - 173.35 ppm (Cq) - 173.95 ppm (Cq)

### PREPARATION B :

### Acide {2-[2-({3-[{[(7S)-3,4-diméthoxybicyclo[4.2.0]octa-1,3,5-trién-7-yl]méthyl} (méthyl)amino]propyl}amino)-2-oxoéthyl]-4,5-diméthoxyphényl}acétique

2,1g de chlorure de thionyle (17,7 mmol, 1,6 éq) est additionné sur une suspension d'acide 2,2'-(4,5-diméthoxybenzène-1,2-diyl)diacétique (2,76g, 10,9 mmol, 1éq) dans le toluène (50 mL). Le milieu réactionnel est porté à 80°C, maintenu 4h sous agitation à cette température. Une surcharge en chlorure de thionyle est ajoutée au milieu réactionnel (519 mg, 4,36 mmol), maintenu 1h puis refroidi à température ambiante.

A cette solution est additionnée à température ambiante de la triéthylamine (3,31 g, 32,7 mmol, 3 éq) en solution dans le dichlorométhane (5 mL), puis une solution de *N*-{[(7*S*)-3,4-diméthoxybicyclo[4.2.0]octa-1,3,5-trién-7-yl]méthyl}-*N*-méthylpropane-1,3-diamine (2,87 g, 10,9 mmol, 1éq) en solution dans le dichlorométhane (10 mL). Après un contact de 30 min, 6 mL d'eau sont additionnés, la phase aqueuse est acidifiée avec une solution d'acide chlorhydrique 1N et extraite avec du dichlorométhane (70 mL). Après mise à sec de la phase organique, le résidu est purifié par flash chromatographie sur colonne de silice (dichlorométhane/méthanol/triéthylamine proportions 80/20/0.1). On obtient 1,92g du produit du titre sous forme d'une meringue beige.

### Rendement : 35%

*RMN ¹H (CDCl₃, 400MHz) :* 1.62 ppm (2H, m) - 2.26 ppm (3H, s) - 2.44 (2H, m) - 2.52 ppm (1H, dd) - 2.65 ppm (1H, dd) - 2.79 ppm (1H, m) - 3.13 ppm (2H, m) -3.18 ppm (1H, dd)-3.44 ppm (2H, m) - 3.46 ppm (2H, s) - 3.49 ppm (2H, s) - 3.72 ppm (2H, m) - 3.73 ppm (3H, s) - 3.74 ppm (3H, s) - 3.77 ppm (3H, s) - 6.59 ppm (1H, s) - 6.63 ppm (1H, s) - 6.67 ppm (1H, s) - 6.72 ppm (1H, s) - 7.62 ppm (NH, t).

*RMN ¹³C (CDCl₃, 100MHz) :* 26.1 ppm (CH₂) - 35.5 ppm (CH₂) - 37.7 ppm (CH₂) - 39.6 ppm (CH₃) - 40.7 ppm (CH₂) - 41.5 ppm (CH) - 42.1 ppm (CH₂) - 55.1 ppm (CH₂) - 55.8 ppm (CH₃) - 55.9 ppm (CH₃) - 56.2 (CH₃) ppm - 56.3 ppm (CH₃) - 60.9 ppm(CH₂) - 106.7 ppm (CH) - 107.4 ppm (CH) - 112.7 ppm (CH) - 113.7 ppm (CH) -126.5 ppm (C q) - 128.6 ppm (C q) - 134.7 ppm (C q) - 137.6 ppm (C q) - 147.6 ppm (C q) -147.8 ppm (C q)-149.5 ppm (C q) - 150.1 ppm (C q) - 172.3 ppm (C q) -178.2 ppm (C q).

### PREPARATION C :

### 3-{3-[{[(7S)-3,4-diméthoxybicyclo[4.2.0]octa-1,3,5-trién-7-yl]méthyl} (méthyl)amino]propyl}-7,8-diméthoxy-1H-3-benzazépine-2,4(3H,5H)-dione

Du chlorure de thionyle (1,68 mmol, 1,68 éq) est introduit sur une suspension d'acide {2-[2-({3-[{[(7*S*)-3,4-diméthoxybicyclo[4.2.0]octa-1,3,5-trién-7-yl]méthyl}(méthyl)amino] propyl}amino)-2-oxoéthyl]-4,5-diméthoxyphényl}acétique (500 mg, 1 mmol) dans le mélange toluène/dichlorométhane (15 ml, 66/33) à 60°C. Après 3h30 de contact, 0,5 mmol de chlorure de thionyle en solution dans 5 mL de dichlorométhane sont ajouté (0.5 éq). Après 1h30 de contact, le milieu réactionnel est refroidi à 25°C. Une solution aqueuse de soude 1N (10 mL) et du dichlorométhane (10 mL) sont ajoutés au milieu. Les deux phases sont séparées, la phase organique est séchée sur sulfate de sodium puis mise à sec. On obtient 0,38g d'une huile rouge foncée. Le produit peut être purifié par chromatographie flash sur colonne de silice (éluant dichlorométhane/méthanol 95/5).

### Rendement : 48 %

*RMN ¹H (CDCl₃ 400MHz):* 1.59 ppm (2H, m) - 1.61 ppm (2H, m) - 2.16 ppm (3H, m) - 2.39 ppm (1H, m) - 2.60 ppm (3H, m) - 3.13 ppm (1H, m) - 3.22 ppm (2H, m) - 3.28 ppm (1H, m) - 3.36 ppm (1H, m) - 3.66 ppm (3H, s) - 3.67 ppm (3H, s) - 3.68 ppm (3H, s) - 3.69 ppm (3H, s) - 3.71 ppm (2H, m) - 6.64 ppm (1H, d) - 6.68 ppm (1H, d) - 6.71 ppm (1H, s)-6.81 ppm (1H, s).
*RMN ¹³C (CDCl₃, 100MHz):* 26.24 ppm (CH₂) - 27.55 ppm (CH₂) - 36.38 ppm (CH₂)-39.16 ppm (CH₂)- 41.54 ppm (CH₂) -41.58 ppm (CH) - 42.90 ppm (CH₃) - 56.54 ppm (CH₂) - 56.56 ppm (CH₃) - 56.70 ppm (CH₃) - 56.98 ppm (CH₃) - 57.03 ppm (CH₃) - 63.15 ppm (CH₂) - 108.63 ppm (CH) - 109.30 ppm (CH) - 115.34 ppm (CH) - 115.73 ppm (CH) - 127.08 ppm (Cq) - 128.15 ppm (Cq) - 136.66 ppm (Cq) - 140.30 ppm (Cq) - 149.60 ppm (Cq) - 149.76 ppm (Cq) - 150.99 ppm (Cq) - 151.57 ppm (Cq) - 173.35 ppm (Cq) - 173.95 ppm (Cq).

### PREPARATION D :

### 7,8-diméthoxy-3-(4-méthoxybenzyl)-1H-3-benzazépine-2,4(3H,5H)-dione

3 g de 7,8-diméthoxy-1*H*-3-benzazépine-2,4(3*H*,5*H*)-dione (12,8 mmol), 2 g de chlorure de 4-methoxybenzyle (12,8 mmol, 1 éq), 2,64 g de K₂CO₃ (19,1 mmol, 1,5 éq), 1,06 g de KI (6,4 mmol, 0,5 éq) et 30 mL d'acétonitrile sont introduits dans un tricol de 100 mL. Le milieu réactionnel est chauffé à 80°C pendant 3h30. Trois fois 1 g de chlorure de 4-methoxybenzyle sont introduits et le milieu réactionnel est maintenu à 80°C pendant 24h. Après retour à température ambiante, 30 mL d'eau et 30 mL de dichlorométhane sont introduits. Après extraction et mise à sec de la phase organique, l'huile rouge foncée obtenue est purifiée par chromatographie flash sur colonne de silice (éluant : dichlorométhane/méthanol 99/1) pour obtenir le produit du titre.

### Rendement : 52%

*RMN ¹H (CDCl₃, 400MHz)* : 3.73 ppm (3H, s) - 3.85 ppm (6H, s) - 4.01 ppm (4H, s) - 4.85 ppm (2H, s) - 6.77 ppm (4H, m) - 7.20 ppm (2H, d).
*RMN ¹³C (CDCl₃ 100MHz):* 44.71 ppm (2 CH₂) - 45.08 ppm (CH₂) - 55.18 ppm (CH₃)-56.11 ppm (2 CH₃) - 111.80 ppm (2 CH) -113.60 ppm (2 CH) -123.64 ppm (2 Cq) - 129.69 ppm (Cq) - 130.01 ppm (2 CH) - 148.75 ppm (2 Cq) - 158.75 ppm (Cq) - 170.82 ppm (2 Cq).

### EXEMPLE 1 :

### 3-{3-[{[(7S)-3,4-diméthoxybicyclo[4.2.0]octa-1,3,5-trién-7-yl]méthyl} (méthyl)amino]propyl}-7,8-diméthoxy-1,3-dihydro-2H-3-benzazépin-2-one

Une solution de 3-{3-[{[(7*S*)-3,4-diméthoxybicyclo[4.2.0]octa-1,3,5-trién-7-yl]méthyl} (méthyl)amino]propyl}-7,8-diméthoxy-1*H*-3-benzazépine-2,4(3*H*,5*H*)-dione (0,47g, 0,97 mmol) dans le THF (10 mL) est refroidie à -78°C. LiBH(Et)₃ (1,3g, 1,46 mmol, 1,5 éq, en solution 1M dans THF) est ensuite additionné lentement sur le milieu réactionnel. Après 1h à -78°C, 15 mL HCl 1N est ajouté sur le milieu. Le milieu réactionnel revient à lentement à température ambiante et est maintenu ainsi pendant 18h. Le produit est extrait avec du dichlorométhane (2 x 10 mL). La phase organique est séchée sur sulfate de sodium puis évaporée. Après purification par flash chromatographie sur colonne de silice (éluant : dichlorométhane/méthanol 9/1), on obtient 180 mg de produit attendu.

### Rendement: 34%

*RMN ¹H (CDCl₃ 400MHz)* : 1.72 ppm (2H, m) - 2.27 ppm (3H, s) - 2.33 ppm (2H, t) - 2.50 ppm (1H, dd) - 2.65 ppm (1H, m) - 2.67 ppm (1H, dd) - 3.21 ppm (1H, dd)- 3.44 ppm (2H, s)- 3.47 ppm (1H, m)- 3.61 ppm (2H, m)- 3.85 ppm (3H, s) - 3.86 ppm (6H, s) - 3.90 ppm (3H, s)- 6.23 ppm (1H, d)- 6.32 ppm (1H, d)- 6.70 ppm (1H, s)- 6.71 ppm (2H, s)- 6.79 ppm (1H, s).

*RMN ¹³C (CDCl₃ 100MHz)* : 26.3 ppm (CH₂) - 35.1 ppm (CH₂) - 40.7 ppm (CH) - 42.4 ppm (CH₃) -43.3 ppm (CH₂) -46.2 ppm (CH₂) -54.7 ppm (CH₂) -55.9 ppm (CH₃) -56.2 ppm (CH₃) -56.3 ppm (CH₃) -61.9 ppm (CH₃) -106.7 ppm (CH) - 107.4 ppm (CH) -109.4 ppm (CH) -111.2 ppm (CH) -117.0 ppm (CH) -124.8 ppm (C q) -126.4 ppm (C q) -128.7 ppm (CH) -135.0 ppm (C q) -139.1 ppm (C q) -148.0 ppm (C q) -149.3 ppm (C q) -149.7 ppm (C q) -149.8 ppm (C q) -167.6 ppm (C q).

### EXEMPLE 2 :

### 3-{3-[{[(7S)-3,4-diméthoxybicyclo[4.2.0]octa-1,3,5-trién-7-yl]méthyl} (méthyl)amino]propyl}-7,8-diméthoxy-1,3,4,5-tétrahydro-2H-3-benzazépin-2-one

Dans un autoclave de 250 mL, 4g de produit obtenu à l'exemple 1 et 2g de Pd(OH)₂ 20% sur charbon (teneur en eau : 50%) sont ajoutés à une solution d'éthanol (90 mL) et d'acide acétique (10 mL). Après 5h de contact à température ambiante sous pression d'hydrogène de 5 bars, le milieu réactionnel est filtré sur Célite ®. Le résidu obtenu après concentration sous pression réduite est repris dans du dichlorométhane (100 mL), puis lavé par une solution aqueuse saturée en bicarbonate de sodium. L'huile obtenue après séchage de la phase organique sur MgSO₄, puis concentration sous pression réduite est purifiée par chromatographie sur silice (dichlorométhane/éthanol/NH₄OH 28% : 95/5/0.5) et 2.6 g de produit du titre sont obtenus sous forme d'huile.

### Rendement : 74%

IR (pur) : σ = 2788, 1646, 1519-1461, 1245-1105 cm⁻¹.

### EXEMPLE 3 :

### 7,8-diméthoxy-3-(4-méthoxybenzyl)-1,3-dihydro-2H-3-benzazépin-2-one

1 g de LiBH(Et)₃ (1,12 mmol, 1,33 éq, solution 1M dans le THF) est additionné sur une solution de 7,8-diméthoxy-3-(4-méthoxybenzyl)-1H-3-benzazépine-2,4(3H,5H)-dione (300mg, 0,84 mmol) dans le THF (4,5 mL) à -78°C. Le milieu réactionnel est maintenu sous agitation 1h30 à cette température puis est hydrolysé par une solution aqueuse saturée de chlorure d'ammonium (4 mL). Après un lent retour à température ambiante, 2 mL d'eau et 5 mL de dichlorométhane sont introduits, la phase aqueuse est extraite par 10 mL de dichlorométhane et la phase organique est mise à sec. Le brut obtenu est purifié par chromatographie flash sur colonne de silice (dichlorométhane/méthanol 98/2) pour obtenir le produit du titre.

### Rendement : 63%

*RMN ¹H (CDCl₃ 400MHz)* : 3.48 ppm (2H,s) - 3.75 ppm (3H, s) - 3.85 ppm (3H, s) -3.89 ppm (3H, s) - 4.67 ppm (2H, s) - 6.18 ppm (1H, d) - 6.29 ppm (1H, d) - 6.68 ppm (1H, s)-6.79 ppm (1H, s) - 6.80 ppm (2H, d) - 7.05 ppm (2H, d).

*RMN ¹³C (CDCl₃, 100MHz) :* 43.20 ppm (CH₂) - 50.18 ppm (CH2) - 55.22 ppm (CH₃) - 55.94 ppm (2 CH₃) - 109.42 ppm (CH) - 111.19 ppm (CH) - 113.94 ppm (2CH) - 117.30 ppm (CH) - 124.63 ppm (Cq) - 126.41 ppm (Cq) - 127.86 ppm (1CH) - 128.65 ppm (Cq)-128.96 ppm (2CH) - 147.95 ppm (Cq) - 149.82 ppm (Cq) - 158.92 ppm (Cq) - 167.90 ppm (Cq).

### EXEMPLE 4 :

### 7,8-diméthoxy-1,3-dihydro-2H-3-benzazépin-2-one

640 mg (1,89 mmol) de 7,8-diméthoxy-3-(4-méthoxybenzyl)-1,3-dihydro-2*H*-3-benzazépin-2-one sont chauffés au reflux dans 4 mL d'acide trifluoroacétique. Après 8h de contact, 8 mL d'eau déminéralisée sont ajoutés et le milieu réactionnel est filtré. Le précipité obtenu est lavé successivement par 4 mL d'eau déminéralisée puis 2 fois 4 mL de méthanol pour obtenir après séchage 401 mg d'une poudre verte correspondant au produit du titre.

### Rendement : 97%

### PF: 236 °C

*RMN ¹H (CDCl₃, 400MHz)* : 3.42 ppm (2H, s) - 3.86 ppm (3H, s) - 3.88 ppm (3H, s) - 6.17 ppm (1H, m) - 6.29 ppm (1H, d) - 6.70 ppm (1H, s) - 6.75 ppm (1H, s) - 7.68 ppm (NH).
*RMN ¹³C (CDCl₃ 100MHz*) *:* 42.65 ppm (CH₂) - 55.97 ppm (2 CH₃) - 109.78 ppm (CH) - 111.48 ppm (CH) - 116.89 ppm (CH) - 122.69 ppm (CH) - 123.56 ppm (Cq) - 126.77 ppm (Cq) - 148.11 ppm (Cq) - 149.87 ppm (Cq) - 170.18 ppm (Cq)/

## Revendications

1. Procédé de synthèse du composé de formule (I): 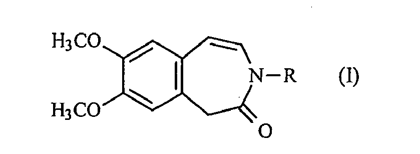 dans laquelle R représente un groupement *para*-méthoxybenzyle (PMB) ou le groupement suivant : 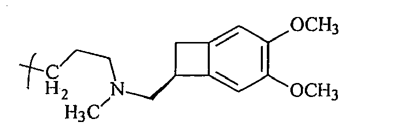 **caractérisé en ce que** le composé de formule (VII): 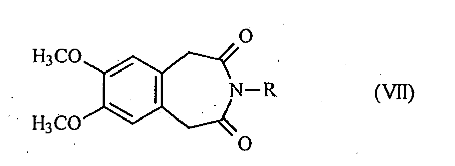 dans laquelle R est tel que défini précédemment,
est soumis à une réaction de réduction,
en présence de LiBH(Et)₃,
dans un solvant organique,
pour conduire au composé de formule (I).

2. Procédé de synthèse selon la revendication 1, **caractérisé en ce que** la quantité de LiBH(Et)₃ utilisée pour effectuer la réaction de réduction du composé de formule (VII) en composé de formule (I) est comprise entre 1 et 3 équivalents.

3. Procédé de synthèse selon l'une quelconque des revendications 1 ou 2, **caractérisé en ce que** le solvant organique utilisé pour effectuer la réaction de réduction du composé de formule (VII) en composé de formule (I) est choisi parmi le tétrahydrofurane (THF), le méthyl-tétrahydrofurane (Me-THF), le dichlorométhane, le toluène ou le diisopropyl éther.

4. Procédé de synthèse selon la revendication 3, **caractérisé en ce que** le solvant organique utilisé pour effectuer la réaction de réduction du composé de formule (VII) en composé de formule (I) est le tétrahydrofurane.

5. Procédé de synthèse selon l'une quelconque des revendications 1 à 4, **caractérisé en ce que** la réaction de réduction du composé de formule (VII) en composé de formule (I) est conduite à une température comprise entre -100 °C et 20 °C.

6. Procédé de synthèse de l'ivabradine de formule (II) selon la revendication 1, dans lequel R représente le groupement suivant : 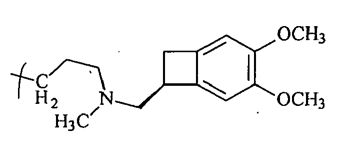 **caractérisé en ce que** le composé de formule (VIII), cas particulier des composés de formule (VII) : 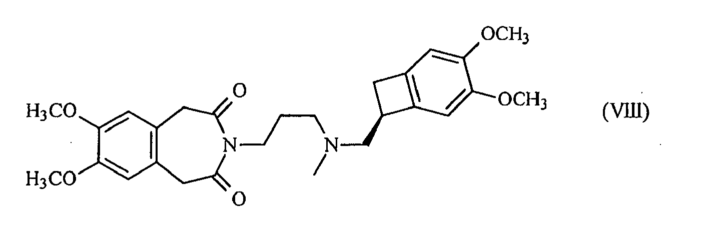 est soumis à une réaction de réduction selon la revendication 1,
pour conduire au composé de formule (VI), cas particulier des composés de formule (I) : 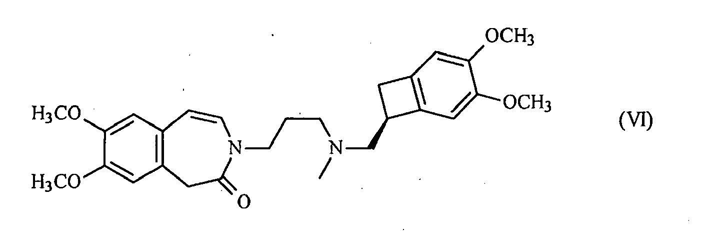 puis le composé de formule (VI) subit une hydrogénation catalytique pour conduire à l'ivabradine de formule (II) : 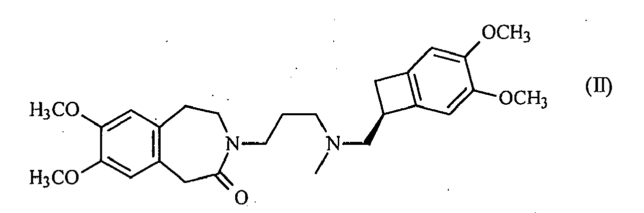 qui peut être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

7. Procédé de synthèse selon la revendication 6, **caractérisé en ce que** le composé de formule (VIII) est préparé à partir du composé de formule (IX) : 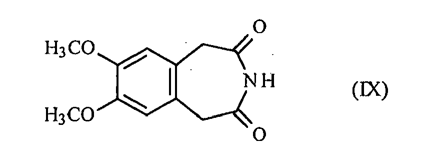 qui est mis en réaction avec le composé de formule (X) : 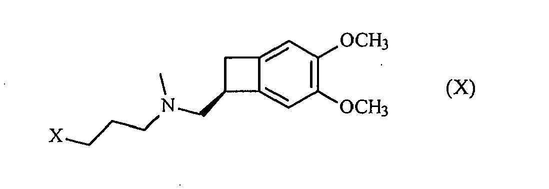 dans laquelle X représente un atome d'halogène, un groupement mésylate ou un groupement tosylate,
en présence d'une base,
dans un solvant organique,
pour conduire au composé de formule (VIII) :

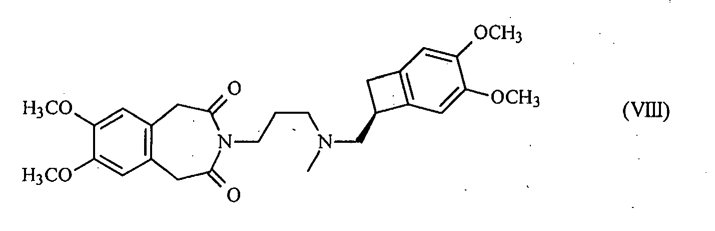

8. Procédé de synthèse selon la revendication 6, **caractérisé en ce que** le composé de formule (VIII) est préparé à partir du composé de formule (XI) : 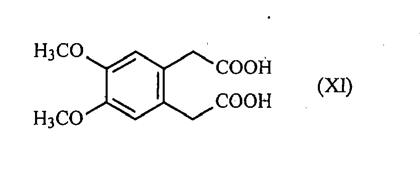 qui est transformé en composé de formule (XII) 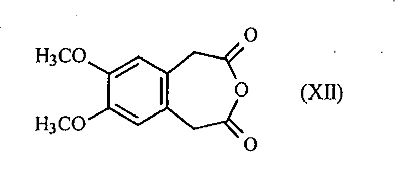 en présence d'un agent de couplage,
dans un solvant organique,
ledit composé de formule (XII) étant ensuite mis en réaction avec le composé de formule (XIII) : 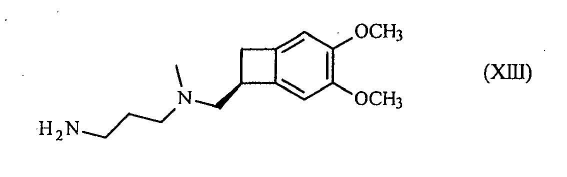 en présence d'une base,
dans un solvant organique,
pour conduire au composé de formule (XIV) : 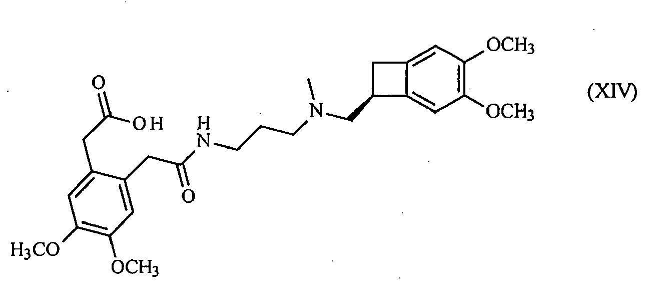 qui est soumis à une réaction de cyclisation en présence d'un agent de couplage,
dans un solvant organique
pour conduire au composé de formule (VIII) :

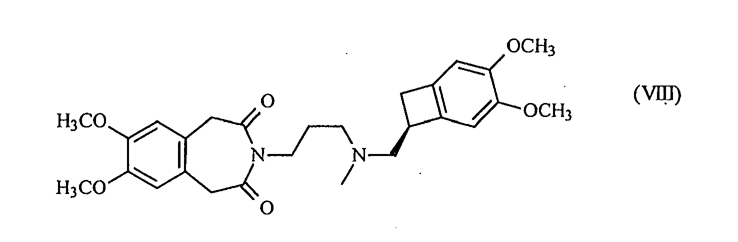

9. Procédé selon la revendication 1, pour lequel R représente un groupement *para-*méthoxybenzyle, pour la préparation du composé de formule (XV) : 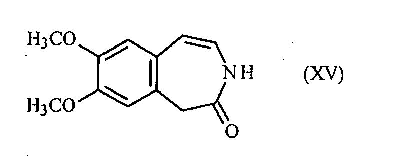 **caractérisé en ce que** le composé de formule (XVI), cas particulier des composés de formule (VII): 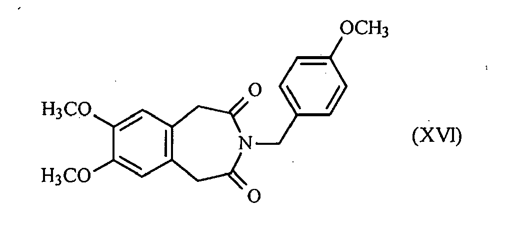 est soumis à une réaction de réduction selon la revendication 1,
pour conduire au composé de formule (XVII), cas particulier des composés de formule (I) : 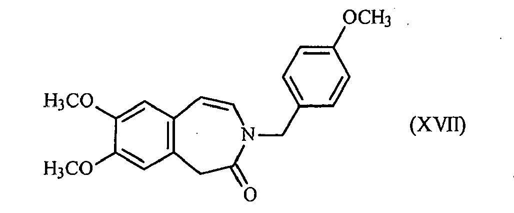 puis le composé de formule (XVII) est déprotégé pour conduire au composé de formule (XV).

10. Composé de formule (VIII) :

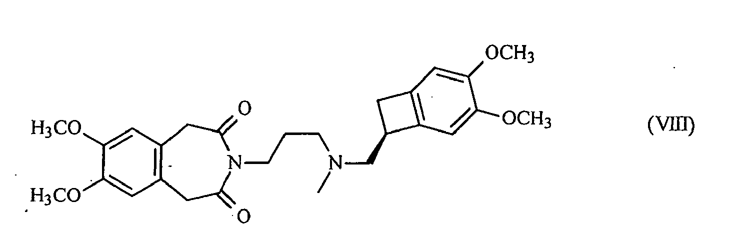

11. Composé de formule (XIV) :

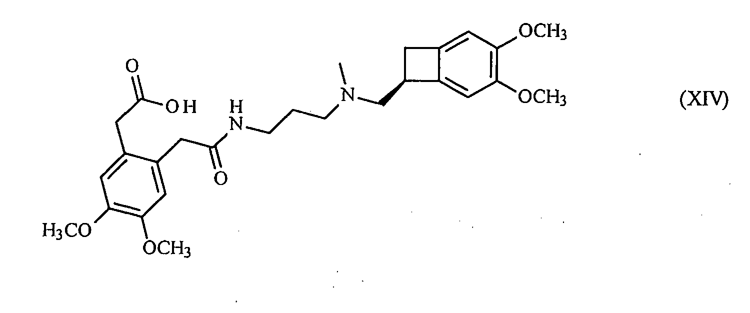

## Patentansprüche

1. Verfahren zur Synthese der Verbindung der Formel (I): 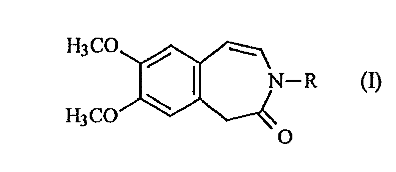 in der R eine *para*-Methoxybenzylgruppe (PMB) oder die folgende Gruppe: 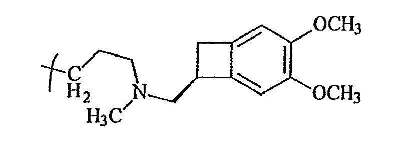 bedeutet, **dadurch gekennzeichnet, dass** die Verbindung der Formel (VII): 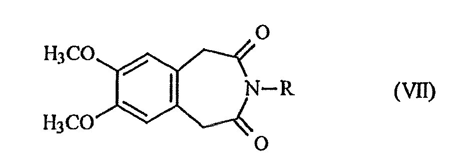 in der R die oben angegebenen Bedeutungen besitzt,
einer Reduktionsreaktion
in Gegenwart von LiBH(Et)₃
in einem organischen Lösungsmittel
unterworfen wird zur Bildung der Verbindung der Formel (I).

2. Syntheseverfahren nach Anspruch 1, **dadurch gekennzeichnet, dass** die zur Durchführung der Reduktionsreaktion der Verbindung der Formel (VII) zu der Verbindung der Formel (I) verwendete Menge LiBH(Et)₃ zwischen 1 und 3 Äquivalenten beträgt.

3. Syntheseverfahren nach einem der Ansprüche 1 oder 2, **dadurch gekennzeichnet, dass** das zur Durchführung der Reduktionsreaktion der Verbindung der Formel (VII) zu der Verbindung der Formel (I) verwendete organische Lösungsmittel aus Tetrahydrofuran (THF), Methyltetrahydrofuran (Me-THF), Dichlormethan, Toluol und Diisopropylether ausgewählt wird.

4. Syntheseverfahren nach Anspruch 3, **dadurch gekennzeichnet, dass** das zur Durchführung der Reduktionsreaktion der Verbindung der Formel (VII) zu der Verbindung der Formel (I) verwendete organische Lösungsmittel Tetrahydrofuran ist.

5. Syntheseverfahren nach einem der Ansprüche 1 bis 4, **dadurch gekennzeichnet, dass** die Reduktionsreaktion der Verbindung der Formel (VII) zu der Verbindung der Formel (I) bei einer Temperatur zwischen -100 °C und 20 °C durchgeführt wird.

6. Verfahren zur Synthese von Ivabradin der Formel (II) nach Anspruch 1, worin R die folgende Gruppe bedeutet: 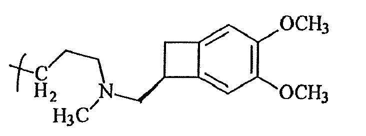 **dadurch gekennzeichnet, dass** die Verbindung der Formel (VIII), ein Sonderfall der Verbindungen der Formel (VII): 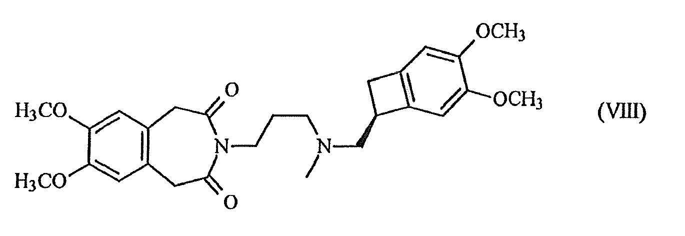 einer Reduktionsreaktion nach Anspruch 1 unterworfen wird,
zur Bildung der Verbindung der Formel (VI), einem Sonderfall der Verbindungen der Formel (I): 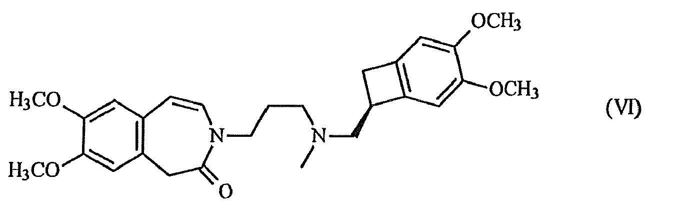 wonach die Verbindung der Formel (VI) einer katalytische Hydrierung unterworfen wird zur Bildung von Ivabradin der Formel (II): 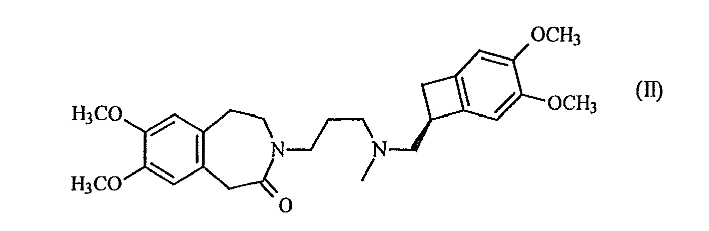 welches in seine Additionssalze mit einer pharmazeutisch annehmbaren Säure ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure, und ihre Hydrate umgewandelt werden kann.

7. Syntheseverfahren nach Anspruch 6, **dadurch gekennzeichnet, dass** die Verbindung der Formel (VIII) ausgehend von der Verbindung der Formel (IX): 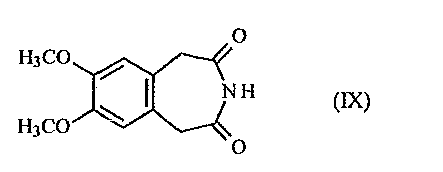 hergestellt wird, welche mit der Verbindung der Formel (X): 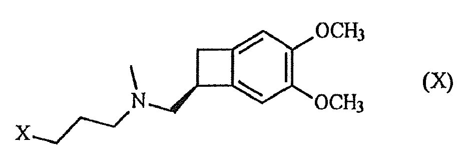 in der X ein Halogenatom, eine Mesylatgruppe oder eine Tosylatgruppe bedeutet,
in Gegenwart einer Base
in einem organischen Lösungsmittel
umgesetzt wird zur Bildung der Verbindung der Formel (VIII):

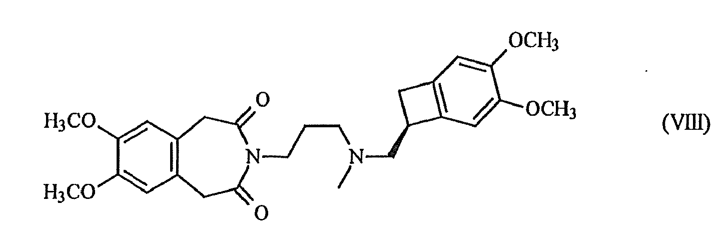

8. Syntheseverfahren nach Anspruch 6, **dadurch gekennzeichnet, dass** die Verbindung der Formel (VIII) ausgehend von der Verbindung der Formel (XI): 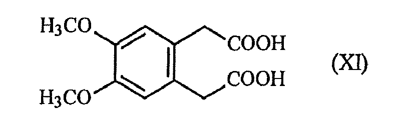 hergestellt wird,
welche in Gegenwart eines Kupplungsmittels
in einem organischen Lösungsmittel
in die Verbindung der Formel (XII): 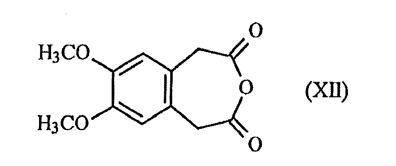 umgewandelt wird,
welche Verbindung der Formel (XII) anschließend mit der Verbindung der Formel (XIII): 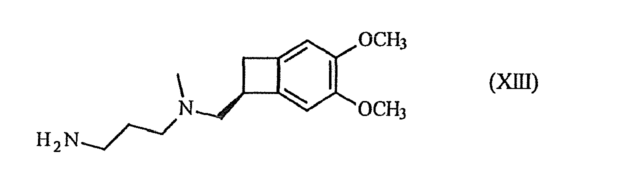 in Gegenwart einer Base
in einem organischen Lösungsmittel umgesetzt wird
zur Bildung der Verbindung der Formel (XIV): 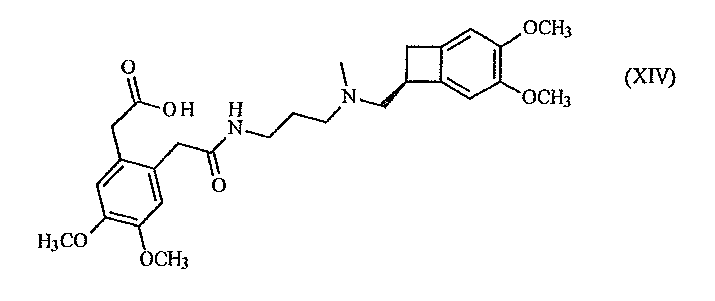 welche in Gegenwart eines Kupplungsmittels einer Cyclisierungsreaktion
in einem organischen Lösungsmittel unterworfen wird
zur Bildung der Verbindung der Formel (VIII):

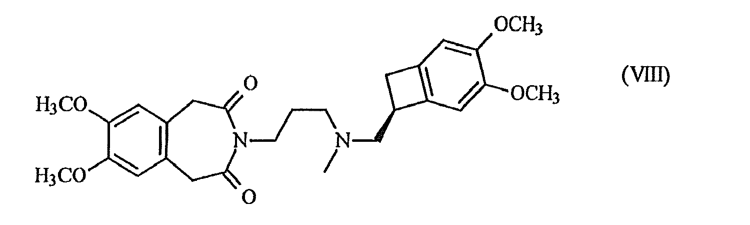

9. Verfahren nach Anspruch 1, worin R eine para-Methoxybenzylgruppe bedeutet, für die Herstellung der Verbindung der Formel (XV): 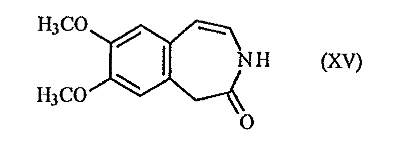 **dadurch gekennzeichnet, dass** die Verbindung der Formel (XVI), ein Sonderfall der Verbindungen der Formel (VII): 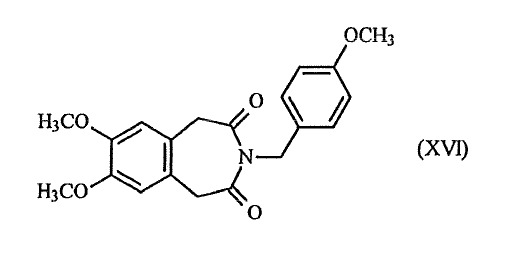 einer Reduktionsreaktion gemäß Anspruch 1 unterworfen wird zur Bildung der Verbindung der Formel (XVII), einem Sonderfall der Verbindungen der Formel (I): 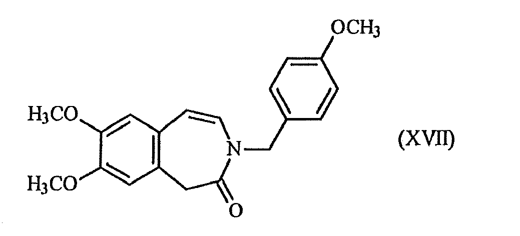 wonach von der Verbindung der Formel (XVII) die Schutzgruppe abgespalten wird zur Bildung der Verbindung der Formel (XV).

10. Verbindung der Formel (VIII):

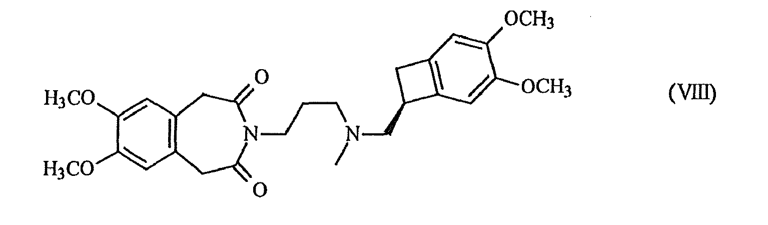

11. Verbindung der Formel (XIV):

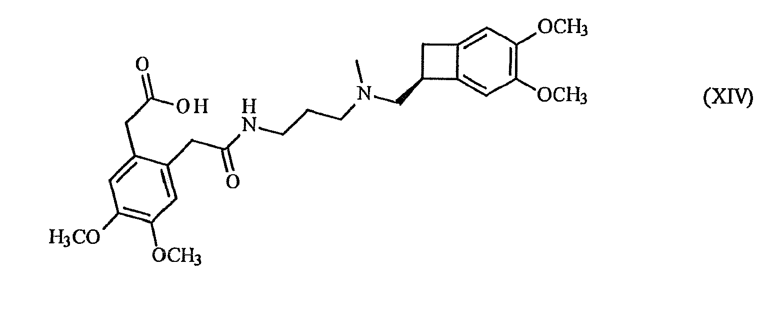

## Claims

1. Process for the synthesis of the compound of formula (I): 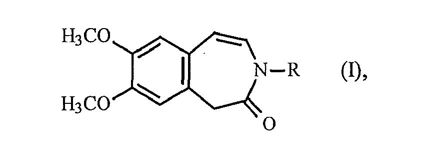 wherein R represents a para-methoxybenzyl (PMB) group or the following group: 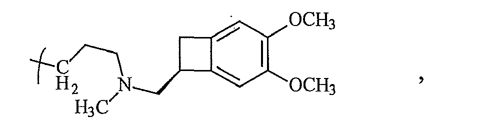 **characterised in that** the compound of formula (VII): 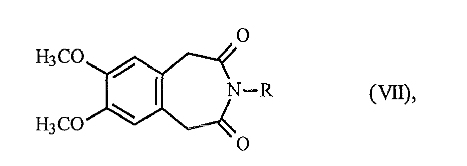 wherein R is as defined hereinbefore,
is subjected to a reduction reaction,
in the presence of LiBH(Et)₃,
in an organic solvent,
to yield the compound of formula (I).

2. Synthesis process according to claim 1, **characterised in that** the amount of LiBH(Et)₃ used to carry out the reduction reaction on the compound of formula (VII) yielding the compound of formula (I) is from 1 to 3 equivalents.

3. Synthesis process according to either claim 1 or claim 2, **characterised in that** the organic solvent used to carry out the reduction reaction on the compound of formula (VII) yielding the compound of formula (I) is selected from tetrahydrofuran (THF), methyl tetrahydrofuran (MeTHF), dichloromethane, toluene and diisopropyl ether.

4. Synthesis process according to claim 3, **characterised in that** the organic solvent used to carry out the reduction reaction on the compound of formula (VII) yielding the compound of formula (I) is tetrahydrofuran.

5. Synthesis process according to any one of claims 1 to 4, **characterised in that** the reduction reaction on the compound of formula (VII) yielding the compound of formula (I) is performed at a temperature from -100 °C to 20°C.

6. Process for the synthesis of ivabradine of formula (II), in accordance with claim 1, wherein R represents the following group: 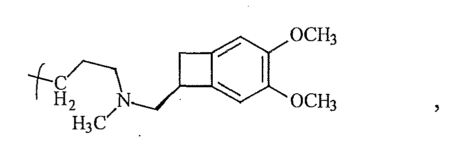 **characterised in that** the compound of formula (VIII), a particular case of the compounds of formula (VII): 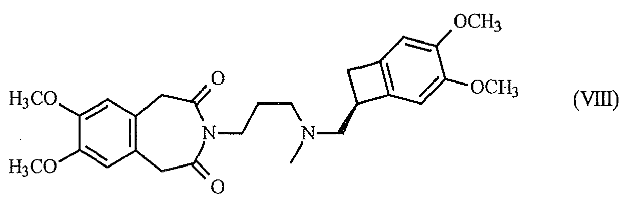 is subjected to a reduction reaction according to claim 1
to yield the compound of formula (VI), a particular case of the compounds of formula (I): 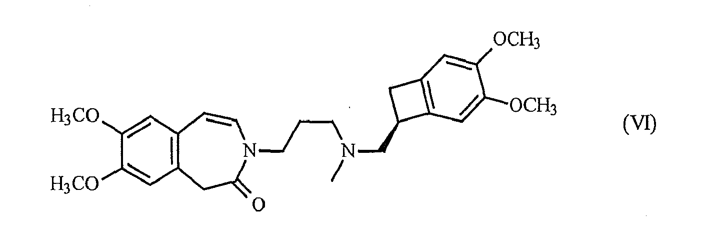 and then the compound of formula (VI) is subjected to catalytic hydrogenation to yield ivabradine of formula (II): 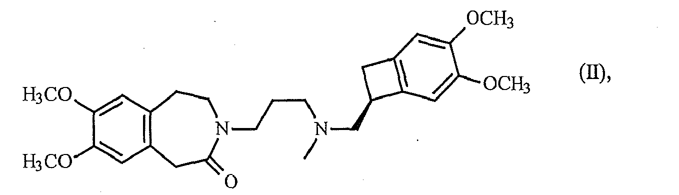 which may be converted into an addition salt thereof with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into hydrates thereof.

7. Synthesis process according to claim 6, **characterised in that** the compound of formula (VIII) is prepared starting from the compound of formula (IX): 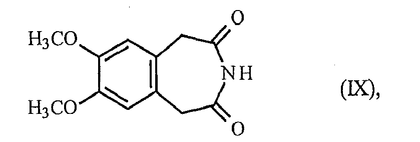 which is reacted with the compound of formula (X): 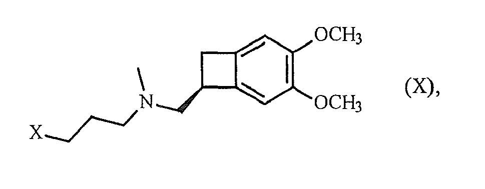 wherein X represents a halogen atom, a mesylate group or a tosylate group,
in the presence of a base,
in an organic solvent,
to yield the compound of formula (VIII):

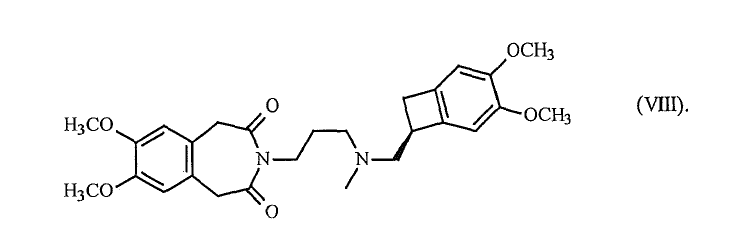

8. Synthesis process according to claim 6, **characterised in that** the compound of formula (VIII) is prepared starting from the compound of formula (XI): 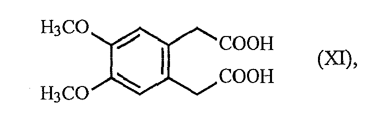 which is converted into the compound of formula (XII): 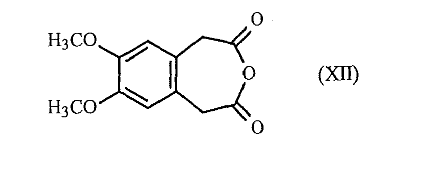 in the presence of a coupling agent,
in an organic solvent,
said compound of formula (XII) then being reacted with the compound of formula (XIII): 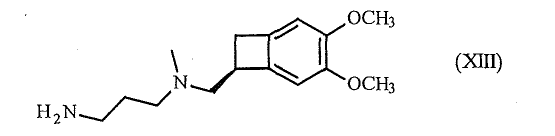 in the presence of a base,
in an organic solvent,
to yield the compound of formula (XIV): 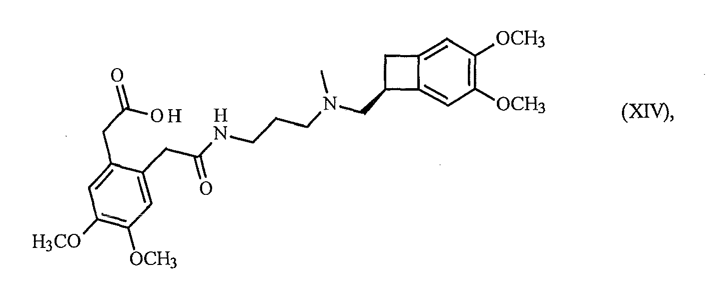 which is subjected to a cyclisation reaction in the presence of a coupling agent,
in an organic solvent,
to yield the compound of formula (VIII):

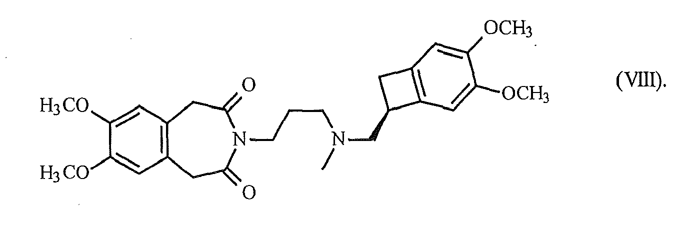

9. Process according to claim 1, wherein R represents a para-methoxybenzyl group, for preparation of the compound of formula (XV): 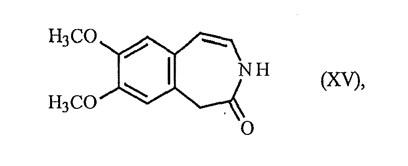 **characterised in that** the compound of formula (XVI), a particular case of the compounds of formula (VII): 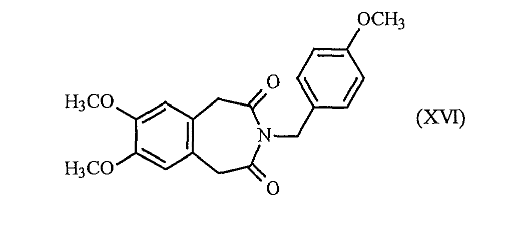 is subjected to a reduction reaction according to claim 1
to yield the compound of formula (XVII), a particular case of the compounds of formula (I): 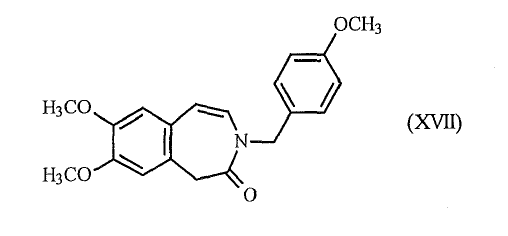 and then the compound of formula (XVII) is deprotected to yield the compound of formula (XV).

10. Compound of formula (VIII):

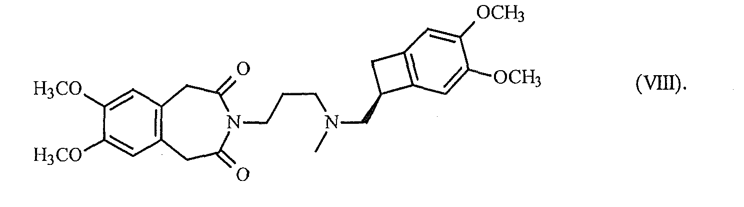

11. Compound of formula (XIV):
